(19) Europäisches Patentamt / European Patent Office / Office européen des brevets

(11) **EP 4 748 493 A1**

(12) # EUROPEAN PATENT APPLICATION

(43) Date of publication:
**27.05.2026 Bulletin 2026/22**

(21) Application number: **24215169.4**

(22) Date of filing: **25.11.2024**

(51) International Patent Classification (IPC):
**B01J 21/06** (2006.01)   **B01J 23/06** (2006.01)
**B01J 35/70** (2024.01)   **B01J 37/02** (2006.01)
**C07C 29/153** (2006.01)

(52) Cooperative Patent Classification (CPC):
(C-Sets available)
**B01J 21/066; B01J 23/06; B01J 35/70;
B01J 37/0203; C07C 29/153**                (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **TOYOTA JIDOSHA KABUSHIKI
KAISHA
Aichi-ken, 471-8571 (JP)**

(72) Inventors:
• **NGUYEN, Phuc Hai
1140 BRUSSELS (BE)**
• **PHONGPRUEKSATHAT, Nat
2628 CD DELFT (NL)**
• **URAKAWA, Atsushi
2628 CD DELFT (NL)**

(74) Representative: **Cabinet Beau de Loménie
103, rue de Grenelle
75340 Paris Cedex 07 (FR)**

Remarks:
Amended claims in accordance with Rule 137(2) EPC.

(54) **HYDROGENATION AND RELATED FLOW REACTOR AND CATALYST AND USE AND PREPARATION THEREOF AND A PRECURSOR USE IN THE PREPARATION**

(57)   A catalyst for synthesizing methanol from carbon dioxide and hydrogen via a catalysis, comprising a zirconium oxide support of a monoclinic crystal structure and catalytic sites provided on the support for the catalysis and from an impregnation with a precursor solution for forming zinc oxide crystals, shows amounts of zinc atoms and zirconium atoms determined on the catalyst by XPS with a ratio of an amount of zinc to an amount of zirconium of 0.4 or more.

Fig. 3

Surface composition by XPS

**(Cont. next page)**

(52) Cooperative Patent Classification (CPC): (Cont.)

C-Sets
**C07C 29/153, C07C 31/04**

**Description**

BACKGROUND OF THE INVENTION

1. Field of the Invention

The invention relates to a method for synthesizing methanol from carbon dioxide and hydrogen, a catalyst for such a method, a flow reactor with and a use of the catalyst and a manufacturing method for the catalyst. The invention also relates to a use of an aqueous solution of dissolved zinc(II)carboxylate as a precursor solution for obtaining the catalyst.

2. Description of Related Art

The reduction of carbon dioxide using hydrogen is a key process in the production of carbon neutral fuels. The synthesis of methanol from carbon dioxide and hydrogen is such a process.

For a low pressure synthesis of methanol from carbon dioxide and hydrogen, catalysts with catalytic sites on an inorganic support are known.

In specific known catalysts, zirconium oxide is used as a support material. In order to form a catalyst this support material is combined with one or more further transition metals by forming a solid solution from zirconium oxide with a source or sources for the one or more further transition metals according to CN 108940254 A, CN 116328779 A, CN 116328773 A or CN 113145113 A. Literatures 1 to 9 show catalysts from co-precipitations from zirconium and zinc nitrate solutions into a homogeneous precipitated precursor (hydrogel of hydroxides of zirconium and zinc). Sites of Zn in a zirconium oxide matrix are formed during the calcination.

Literature 9 shows a co-precipitation with zinc nitrate, zirconium oxy-nitrate, and urea from a heated solution with subsequent aging for more than 12 hours in order to obtain a catalyst for synthesizing methanol from carbon dioxide.

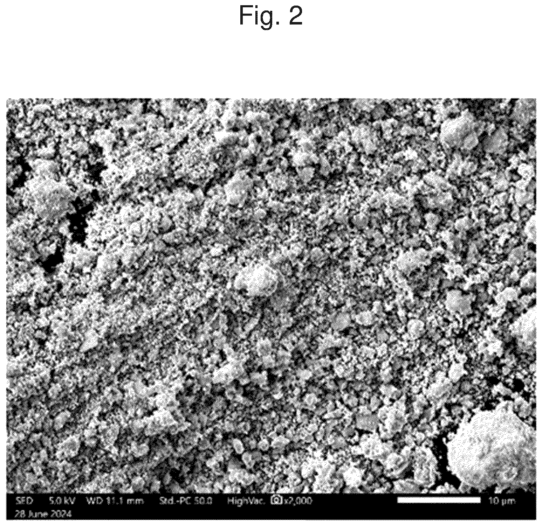

A different concept of combining zirconium oxide with a source of zinc in order to obtain a catalyst is schematically illustrated in FIG. 2 with a structure which provides zinc oxide crystals as catalytic sites on a zirconium oxide support. Such a concept is shown in Literatures 10 and 11 with an impregnation of a zirconium oxide support material with an aqueous solution of zirconium nitrate.

Generally, and in particular in Literatures 10 and 11, significantly high pressures (like 5 MPa or 5 bar) and temperatures in a fixed bed reactor with a catalyst have been recommended for synthesizing methanol from carbon dioxide and hydrogen which are typically provided together in a reactor. The gas swing operation is another synthesizing approach known in chemical engineering, wherein a first and a second gas to be reacted with each other are provided to a catalyst as separate gas streams, a gas stream of the second gas replacing a gas stream of the first gas, e.g. after a purging operation with, e.g., an inert gas. The gas swing operation requires specific operating parameters, including gas concentrations, temperatures, and cycling periods. Generally, the formation and dispersion of Zn, in particular as ZnO particles over $ZrO_2$ is a complex task but important to provide active sites for activation of $CO_2$ and $H_2$ for reducing carbon dioxide. The active sites must be suitable for formation of a target product like methanol. For synthesizing methanol from carbon dioxide and hydrogen synthesis parameters which can be found in Literatures 1 through 11 include pressures between 2 and 5 MPa, temperatures between 230 and 700 °C, $CO_2$ conversions between 0 and 24%, and methanol selectivities between 0 and 100%.

Literature:

1 Xu et al., Highly dispersed metal doping to ZnZr oxide catalyst for CO2 hydrogenation to methanol: Insight into hydrogen spillover, J. Catal., 393, nan, 2021, https://doi.org/10.1016/j.jcat.2020.11.039
2 Lee et al., Atomic Pd-promoted ZnZrOx solid solution catalyst for CO2 hydrogenation to methanol, Appl. Catal. B Environ., 304, nan, 2022, https://doi.org/10.1016/j.apcatb.2021.120994
3 Feng et al., Asymmetric Sites on the ZnZrOx Catalyst for Promoting Formate Formation and Transformation in CO2 Hydrogenation, J. Am. Chem. Soc., 145, 23.0, 2023, https://doi.org/10.1021/jacs.3c02248
4 Hussain et al., First principles investigations of structural and electronic properties of Ga-doped ZnZrOx solid solutions for catalytic reduction of CO2, Mol. Cat., 537, nan, 2023, https://doi.org/10.1016/j.mcat.2023.112941
5 Temvuttirojn et al., Role of Calcination Temperatures of ZrO2 Support on Methanol Synthesis from CO2 Hydrogenation at High Reaction Temperatures over ZnOx/ZrO2 Catalysts, Ind. Eng. Chem. Res., 59, 13.0, 2020, https://doi.org/10.1021/acs.iecr.9b05691
6 Redekop et al., Zn Redistribution and Volatility in ZnZrOx Catalysts for CO2 Hydrogenation, Chem. Mater., 35, 24.0, 2023, https://doi.org/10.1021/acs.chemmater.3c01632

7 Sha et al., The role of surface hydroxyls on ZnZrOx solid solution catalyst in CO2 hydrogenation to methanol, Chin. J. Catal., 45, nan, 2023, https://doi.org/10.1016/S1872-2067(22)64176-7

8 Mao et al., The lattice oxygen determines the methanol selectivity in CO2 hydrogenation over ZnZrOx catalysts, Catalysis Science & Technology, 14, 2.0, 2023, https://doi.org/10.1039/d3cy01471a

9 Wang et al., A highly selective and stable ZnO-ZrO2 solid solution catalyst for CO2 hydrogenation to methanol. Sci Adv. 2017 Oct 6;3(10):e1701290. doi: 10.1126/sciadv. 1701290

10 Zou et al., Design of technical ZnO/ZrO2 catalysts for CO2 hydrogenation to green methanol, J. Catal. 2024, 430, 115344

11 Nikolajsen et al., Surface ZnOX on zirconia is highly active for high temperature methanol synthesis , J. Catal. 2024, 431, 115389

With prior art catalysts and methods with regard to aspects of synthesizing methanol from carbon dioxide and hydrogen, improvement is desired, with regard to, singly or in combinations:

- energy efficiency,
- cost efficiency in obtaining a catalyst,
- catalyst performance, in particular at moderate pressure, in particular below 1 MPa,
- methanol productivity,
- methanol production rate,
- selectivity in synthesizing methanol from carbon dioxide and hydrogen.

SUMMARY OF THE INVENTION

It is an object of the invention to improve efficiency of the synthesis of methanol from carbon dioxide and hydrogen at moderate temperature and pressure conditions. It is an object of the invention to allow an excellent catalyst performance in the synthesis of methanol from carbon dioxide and hydrogen. It is an object of the invention to improve energy efficiency of the synthesis of methanol from carbon dioxide and hydrogen. It is an object of the invention to offer a catalyst for the synthesis of methanol from carbon dioxide and hydrogen which is obtainable in an efficient, in particular cost-efficient manner. It is an object of the invention to address aforementioned problems or drawbacks of the prior art.

An aspect of the invention is a use of an aqueous solution of dissolved zinc(II)carboxylate for producing a catalyst for synthesizing methanol from carbon dioxide and hydrogen via a catalysis, comprising a zirconium oxide support and catalytic sites provided on the support for the catalysis and from an impregnation with a precursor solution for providing the dissolved zinc(II)carboxylate and forming zinc oxide crystals. The material of the zirconium oxide support preferably has a monoclinic crystal structure. Thus, monoclinic $ZrO_2$ may be the support, and the zinc oxide crystals are preferably dispersed thereon as particles having a hexagonal crystal structure.

An aspect of the invention is a method of producing the catalyst and the aspect of the invention being the use of an aqueous solution is preferably a use in this method. This method comprises steps of

providing a dry material, e.g. a powder, of the zirconium oxide support,

impregnating the dry material with a precursor for providing catalytic sites of zinc via impregnation in which zinc is associated and/or interacts with an organic component, the precursor being preferably an aqueous solution of a carboxylic acid salt, more preferably a formate, an acetate, a propionate, an isopropionate, a butyrate, or an isobutyrate, most preferably an acetate, of zinc, preferably with a zinc concentration of 12 mol% to 25 mol%, more preferably 15 mol% to 20 mol%, and preferably with an operation of dropping the aqueous solution at 0.5 to 1 ml/min onto the support heated to a temperature between 80 and 100 °C, and more preferably also with performing the operation of dropping alternately with an operation of grinding for obtaining a powder, preferably with a size between 40 and 80 mesh, and

removing solvent from the aqueous solution, e.g., with a drying step, and then preferably calcining.

An exemplary sequence of steps for processing materials into a catalyst according to the invention comprises the following steps 1 to 6:

step 1 - drying monoclinic zirconium oxide (m-$ZrO_2$) overnight at 120 °C to obtain a dry white powder as a support material,

step 2 - preparing an aqueous solution of 0.5 to 3 mmol/mL $Zn(CH\square CO\square)\square$,

step 3 - heating the product from step 1 between 80 °C and 100 °C to obtain a dry white powder,

step 4 - slowly dropping the solution from step 2 observing a dropping speed of 0.5 and 1 ml / second, using a dropper and alternately with grinding using a pestle to obtain a homogeneous mixed powder dry white powder,

step 5 - evaporating water, e.g., using an oven evaporator at 80 °C to 100 °C and atmospheric pressure for 12 to 10 h, to obtain a dried, white powder,

step 6 - grinding and then calcining the powder from step 5 in a muffle furnace heated at e.g. 10 °C/min to a temperature between 400 °C and 600 °C and keeping this temperature for 3 to 5 hours to obtain a white powder.

An aspect of the invention is a catalyst for synthesizing methanol from carbon dioxide and hydrogen via a catalysis, comprising a zirconium oxide support and catalytic sites provided on the support for the catalysis and from an impregnation with a precursor solution for forming zinc oxide crystals, which catalyst may comprise 1 weight% or more and 30 weight%, or less of zinc oxide crystals as the catalytic sites based on the total weight of the final catalysts with the catalytic sites provided on the support and is preferably conditioned with a hydrogen pretreatment, and the catalyst of the invention has at least one of the following features a) to c):

a) being obtainable according to the aspect of the invention which is the method of producing the catalyst,
b) amounts of zinc atoms and zirconium atoms determined on the catalyst by XPS (x-ray photoelectron spectroscopy) in accordance with a ratio of an amount of zinc to an amount of zirconium of 0.4 or more, preferably 0.6 or more, more preferably 0.7 or more, most preferably 0.8 or more, preferably with an upper limit of 1.5 or less, more preferably 1.2 or less, most 1.1 or less, and
c) in an X-ray diffraction measurement, a ratio of an intensity of a peak attributable to zinc oxide crystals having a hexagonal crystal structure and in a range of Bragg angles between $2\theta = 35°$ and $2\theta = 38°$ to an intensity of a peak attributable to monoclinic zirconium oxide crystals and in a range of Bragg angles between $2\theta = 40°$ and $2\theta = 43°$, which ratio is 1.7 or more.

A catalyst according to the invention, in particular one obtained with the above exemplary sequence of steps for processing materials into a catalyst, may be subjected to the following sequence of steps to be pretreated with hydrogen (converted into a conditioned catalyst) and used in a gas swing operation for obtaining methanol:

| Step | Conditions (ranges) |
| --- | --- |
| Pretreatment with $H_2$ | Flow rate: 5 - 50 mL/min<br>Time: 0 -120 min<br>Temperature: 100 - 300 °C |
| $CO_2$ feed | $CO_2$: 0.01 - 100%<br>Flow rate: 5 - 50 mL/min<br>Time: 5 - 30 min<br>Temperature: 200 - 300 °C |
| Purge with He | Flow rate: 5 - 50 mL/min<br>Time: 0 - 7 min |
| $H_2$ feed | $H_2$: 10 - 100%<br>Flow rate: 5 - 50 mL/min<br>Time: 10 - 60 min<br>Temperature: 200 - 300 °C |
| Purge with He | Flow rate: 5 - 50 mL/min<br>Time: 0 - 7 min |

The purging with He before and/or after the hydrogen feeding step may be performed without heating or with the temperature of 200 to 300 °C of the hydrogen feeding step. Preferably a constant temperature is used from starting the carbon dioxide feeding step until the end of the hydrogen feeding step.

An aspect of the invention is also a flow reactor for synthesizing methanol from carbon dioxide and hydrogen comprising the catalyst according to the invention.

According to an aspect of the invention a catalyst for synthesizing methanol from carbon dioxide and hydrogen via a catalysis, in particular the catalyst of the invention, is used in a gas swing operation. Pertaining further aspects of the invention are a method and a use of a catalyst for synthesizing methanol from carbon dioxide and hydrogen via a catalysis, in particular the catalyst according to the invention, in this method, which is a method for synthesizing methanol from carbon dioxide and hydrogen via a catalysis specified below and with gas flows over the catalyst including a gas flow including the hydrogen and a flow including carbon dioxide established before the gas flow including the hydrogen.

Preferably, the catalysis proceeds at a temperature between 180 °C and 400 °C, more preferably between 200 °C and 350 °C, and a pressure of less than 1.5 MPa and more preferably 1.0 MPa or less. Preferably, a selectivity to methanol in the synthesizing methanol is 40% or more, particularly preferably 60% or more, most preferably 80% or more. A methanol productivity (conversion) with the invention's catalyst and in the invention's method for synthesizing methanol is preferably 0.5 mg per g catalyst and per hour, more preferably 0.6 mg per g catalyst and per hour in an evaluation identified below in the detailed description.

The invention's method for synthesizing methanol from carbon dioxide and hydrogen via a catalysis in the flow reactor, comprises steps of

establishing in the flow reactor a flow of a gas containing the carbon dioxide for an interaction between the carbon dioxide and the catalyst, in preferably with a concentration of 20 vol% or lower, more preferably 15 vol% or lower, particularly preferably 10 vol% or lower in an inert gas or mixture of inert gases like e.g. nitrogen or preferably helium, and at a temperature between preferably 180 °C and 400 °C, more preferably 200 °C and 325 °C, most preferably between 250 °C and 280 °C, this step being preferably followed by purging with inert gas,

replacing in the flow reactor the flow of the gas containing the carbon dioxide by a flow of a gas containing the hydrogen and synthesizing methanol in the flow reactor with the flow of a gas containing the hydrogen, preferably in an amount between 10 weight% and 100 weight%, and at a temperature between preferably 180 °C and 400 °C, more preferably between 200 °C and 325 °C, most preferably between 250 °C and 280 °C.

The above flow containing carbon dioxide may also be one containing 400 ppm or less, preferably 200 ppm or less of the carbon dioxide in the inert gas. A lower limit of the concentration of carbon dioxide is preferably 10 ppm, more preferably 100 ppm. The catalysis proceeds at a temperature preferably between 180 °C and 400 °C, more preferably between 200 °C and 325 °C, most preferably between 250 °C and 280 °C, and a pressure of less than 1.5 MPa, more preferably 1.0 MPa or less, this step being preferably followed by purging with inert gas. Flow velocities of the gas flows (preferably from the gas flow containing carbon dioxide to a purging gas flow following the hydrogen containing gas flow) are preferably between 10 and 50 ml/min.

A particular aspect of the invention, specifically of the above-mentioned method and/or catalyst use for synthesizing methanol from carbon dioxide and hydrogen, is a process which integrates $CO_2$ capture and methanol production so as to benefit in particular from the catalyst's capability to interact with carbon dioxide. Then one production site and/or facility serves for the capture and subsequent conversion of carbon dioxide to produce methanol with reduced effort in terms of regeneration, equipment, energy and/or transport of gas and/or the product. In particular, under this aspect, a loop process can be formed which generates $CO_2$ from methanol in a conventional manner (e.g., combustion) for obtaining energy and reuses the $CO_2$ to store energy with the synthesis of methanol according to the invention. Accordingly, an aspect of the invention is also an energy buffer.

Obtainable advantages

The inventors examined an impregnation to establish catalytic sites with the metal of a salt and found in particular an impregnation with regard to the impregnation concept shown in Literature 11. Unexpectedly the inventors found an impact of the nature of a precursor solution, in particular the kind of metal salt used in an impregnation to establish catalytic sites with the metal of the salt, on characteristics, in particular a performance of a catalyst obtained via such an impregnation. The finding is believed to relate to a significant change in the catalytic mechanisms used in the prior art like Literature 11. In line with above-mentioned aspects of the invention the inventors found a significant beneficial modification of the prior art in

- a use of a particular precursor solution,
- combining the resulting catalyst with a gas swing operation for carbon dioxide hydrogenation, and/or
- thus efficiently, and in particular with high methanol selectivity, achieving carbon dioxide hydrogenation, particularly with moderate pressure and/or carbon dioxide concentration in a flow reactor (in contrast to a common knowledge-based expectation of less methanol productivity upon lowering a pressure in carbon dioxide hydrogenation).

According to a particular option, the invention allows obtaining a catalyst from monoclinic $ZrO_2$ and an aqueous solution with dissolved zinc(II)carboxylate like zinc acetate so as to work without further transition metal elements and/or chelating agent, with moderate cost, with a moderate environmental impact and/or moderate impact on generated waste water quality. The invention provides a catalyst suitable for an excellently productive gas swing operation and e.g. at a pressure of 1 MPa or less and e.g. with a first gas flow of 10% $CO_2$ in an inert gas like $N_2$, He or Ar, preferably $N_2$, and further separate gas flow of $H_2$, e.g. 100% $H_2$. Thus, the invention offers cost efficiency also in terms of energy and equipment used in synthesizing or in a catalysis for synthesizing methanol. It is believed that this efficiency results from more methanol-favored surface species like formates formed during $CO_2$ feeding and that can be used completely for reaction with $H_2$ and from accessibility of catalytic sites on the invention's catalyst. The invention offers a simple synthesis procedure of catalysts and a high methanol productivity and selectivity at moderate effort.

BRIEF DESCRIPTION OF THE DRAWINGS

Features, advantages, and technical and industrial significance of exemplary embodiments of the invention will be described below with reference to the accompanying drawings, wherein:

FIG. 1A shows a comparison of XRD (x-ray diffraction) patterns from a catalyst resulting from a co-precipitation (CP) with a catalyst resulting from an impregnation of a support (IMP);
FIG. 1B shows a comparison of XRD patterns from catalysts from an impregnation of a support (IMP) with different precursor solutions;
FIG. 2 shows an SEM image from the catalyst resulting from an impregnation of a support (IMP) used for recording the respective spectrum shown in FIG. 1A;
FIG. 3 shows a comparison of amounts determined based on XPS (x-ray photoelectron spectrometry) of atoms on the catalysts the XRD patterns of which are shown in FIG. 1B,
FIGS. 4A to 4C show schematically time resolved component measurement profiles in a method according to the invention;
FIG. 5 shows measurement tuples with employed temperatures and obtained methanol selectivities for gas swing and constant flow operations with a catalyst.

DETAILED DESCRIPTION AND EXAMPLES

Preparation of a comparative sample (CP/ZnO)

A catalyst in line with above Literature 9 was prepared with the following sequence of steps and products resulting from the respective steps:

| Step | | Product |
|---|---|---|
| 1 | heating 1000 ml water in a round-bottom flask at 50 °C | |
| 2 | dissolving<br>1.657 g of $Zn(NO_3)_2 \cdot 6H_2O$,<br>12.6566 g of $ZrO(NO_3)_2 \cdot 6H_2O$,<br>25.6241 g of $CO(NH_2)_2$. | solution of $Zn^{2+}_{(aq)}$, $ZrO^{+2}$, $NO_3^-_{(aq)}$, and $CO(NH_2)_2$, urea transparent solution |
| 2 | stirring and heating under reflux at 90 °C in an oil bath for 24 hours. | opaque suspension (white) |
| 3 | filtering the precipitate using a Buchner funnel | wet, white translucent power |
| 4 | evaporating water using an oven evaporator at 40-60 °C and atmospheric pressure overnight (around 12 - 14 h) | dried, white powder |
| 5 | calcining the grinded powder from step 5 in a muffle furnace at 500 °C, $\beta$ = 10 °C/min for 3 h (stagnant air.) | white powder |

Preparation of a sample according to the invention (IMP/acetate) and further samples, not according to the invention ($ZrO_2$, nitrate, chloride)

A catalyst was prepared with the following sequence of steps and products resulting from the respective steps:

| Step | | Product |
|---|---|---|
| 1 | drying m-$ZrO_2$ (m = monoclinic phase, from Alfa Aesar #43815) overnight at 120 °C. | dry white powder |

(continued)

| Step | | Product |
|---|---|---|
| 2 | dissolving 3.03 g of $Zn(CH_3CO_2)_2$, (99.99% purity with regard to trace metal-based impurity from Sigma-Aldrich #383317) in 10 ml deionized water | 17 mol% solution of $Zn^{2+}_{(aq)}$, having $CH_3CO_2^-_{(aq)}$ as a counter ion and being a transparent solution |
| 3 | heating the $m$-$ZrO_2$ support at 100 °C on an evaporating dish. | dry white powder |
| 4 | slowly dropping the $Zn(CH_3CO_2)_2$ solution from step 2 onto the heated material from step 3 using a dropper alternately with grinding using a pestle | homogeneous mixed powder, dry white powder |
| 5 | evaporating water using an oven evaporator at 100 °C and atmospheric pressure overnight (around 12-14 h) | dried, white powder |
| 6 | calcining grinded powder from step 5 in a muffle furnace at 500 °C, $\beta$ = 10 °C/min for 3 h, stagnant air | white powder |

The product obtained directly from step 1 is taken as a sample labelled $ZrO_2$. The product obtained from step 6 is one according to the invention and forms a sample labelled IMP or acetate. Above steps 1 to 6 were repeated with a replacement of the zinc salt in step 2 so as to produce in step 2 a 17 mol% solution of $Zn^{2+}_{(aq)}$, having Cl- or $NO_3^-$, respectively, as a counter ion and the products then obtained from step 6 form a sample labelled chloride (from the use of Cl as the counter ion in step 2) and a sample labelled nitrate (from the use of $NO_3^-$ as the counter ion in step 2).

XRD, SEM, and XPS measurements

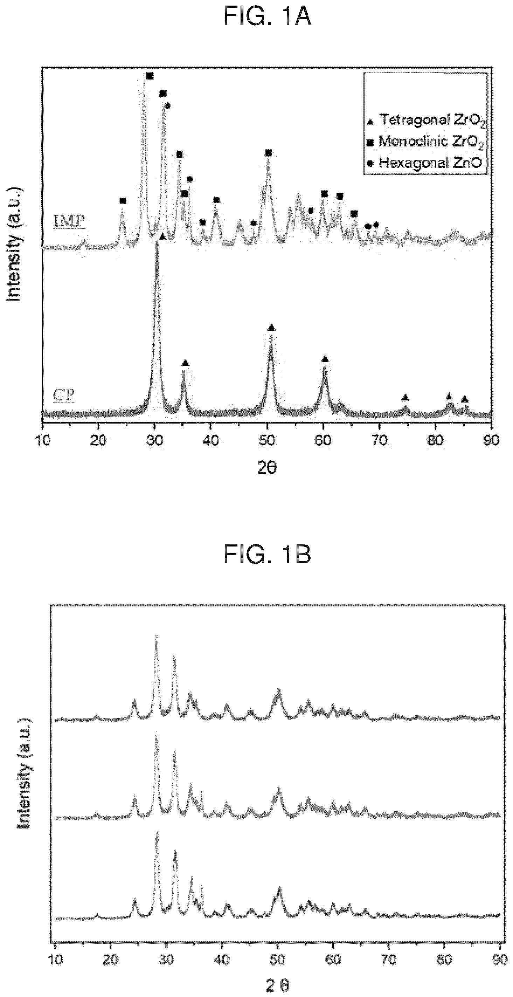

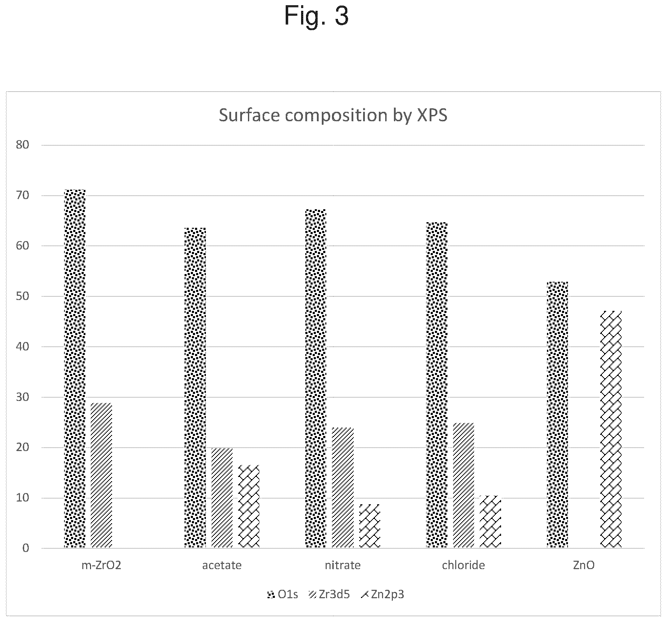

An XRD (x-ray diffraction) measurement like e.g. described in above Literature 9 (section 'Catalyst characterization'), SEM (scanning electron microscope) imaging, and XPS (x-ray photoelectron spectroscopy) are commonly known techniques with commercial measurement devices. For the above samples CP and IMP (also labelled acetate) measured XRD patterns are shown in FIG. 1A, wherein and triangle, square and circle symbols are marking peaks attributable to crystal species listed in the legend in FIG. 1A. For the above samples, acetate (also labelled IMP), chloride and nitrate, measured XRD patterns are shown in FIG. 1B, wherein that of the sample acetate is the lowermost, that of the sample chloride is the uppermost, and that of the sample nitrate is indicated between those of the samples acetate and chloride. In FIG. 1A and 1B the sample IMP/acetate shows a peak attributable to zinc oxide crystals having a hexagonal crystal structure and in a range of Bragg angles between $2\theta = 35°$ and $2\theta = 38°$, which is absent or of a lower intensity in case of the other samples shown in FIG. 1A and 1B. Specifically in FIG. 1B a ratio of an intensity of a peak attributable to zinc oxide crystals having a hexagonal crystal structure and in a range of Bragg angles between $2\theta = 35°$ and $2\theta = 38°$ to an intensity of a peak attributable to monoclinic zirconium oxide crystals and in a range of Bragg angles between $2\theta = 40°$ and $2\theta = 43°$ is a ratio of 1.7 or more. FIG. 2 shows an SEM image of the sample IMP/acetate, wherein the white bar in the right portion of the bottom under the micrograph indicates a scale of 10 $\mu$m. FIG. 3 shows amounts in atomic % for a set of atoms consisting of O, measured based on binding energy of O1s, Zn, measured based on binding energy of Zn2p3, and Zr, measured based on binding energy of Zr3d5, and for each of the aforementioned samples.

Performance evaluation and measurement

A respective sample was placed in a tubular fixed-bed reactor as described in the section 'Catalyst evaluation' of Literature 9 and subjected to the following sequence of gas flows at a pressure of 1 MPa:

| Gas flow | Details | start time | end time |
|---|---|---|---|
| Pretreatment | $H_2$, 40 mL/min, 60 min 300 °C | | |
| $CO_2$ feed | $CO_2$ 3 vol% in He, 40 mL/min, 20 min , 300°C | $t_0$ | $t_1$ |
| Purge | He, 40 mL/min, 5 min, 300°C | $t_1$ | $t_2$ |
| $H_2$ feed | $H_2$, 40 mL/min, 40 min, 300°C | $t_2$ | $t_3$ |
| Purge | He, 40 mL/min, 5 min, no heating | $t_3$ | $t_4$ |

The fixed-bed reactor is equipped with an in-line IR spectrometer and related analysis and monitoring system to detect chemical component concentrations in a known manner, to convert the component concentrations into molar fractions $y_i$ for each respective chemical component i, and to obtain molar flows $F_i$ [mol/min] for each respective chemical component i according to the following equation:

$$F_i = y_i \times \frac{v_T}{22400 \ ml/mol}$$

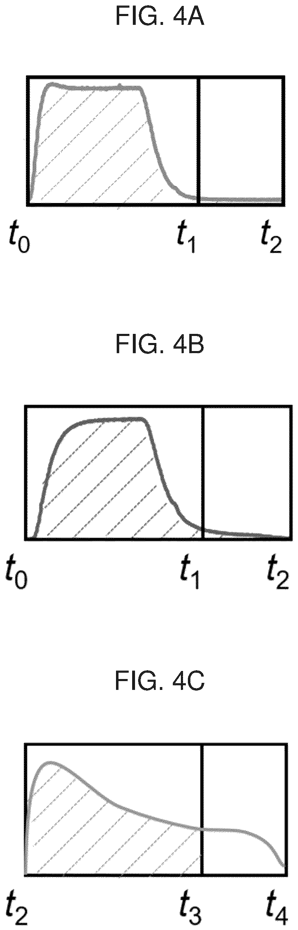

In each of FIGS. 4A to 4C the ordinate indicates correspondingly obtained molar flows $F_i$ and the abscissa indicates a time scale on which above-mentioned start and end times $t_0$ to $t_4$ are indicated. Specifically in an exemplary and schematic manner

- FIG. 4A indicates a profile of a molar flow $F_i$, wherein the chemical component i is $CO_2$ and the sample in the reactor is the sample $ZrO_2$,
- FIG. 4B indicates a profile of a molar flow $F_i$, wherein the chemical component i is $CO_2$ and the sample in the reactor is the sample acetate, and
- FIG. 4C indicates a profile of a molar flow $F_i$, wherein the chemical component i is one of $CH_4$, $CH_3OCH_3$, $CH_3OH$, and $CH_4$, and the sample in the reactor is the sample acetate,
  and thus
- the hatched area in FIG. 4A divided by the weight of the sample $ZrO_2$ used in the reactor represents the $CO_2$ feed amount from $t_0$ to $t_2$ per sample weight ($A_{CO2}$ [mol/g (cat.)]) and in the step of establishing in the reactor a flow of a gas containing the carbon dioxide for an interaction between the carbon dioxide and the catalyst,
- the hatched area in 4B divided by the weight of the sample acetate used in the reactor represents the $CO_2$ feed amount from $t_0$ to $t_2$ per sample weight not captured in the step of establishing in the flow reactor a flow of a gas containing the carbon dioxide for an interaction between the carbon dioxide and the catalyst (sample acetate), and by subtracting this not captured amount per sample weight from $A_{CO2}$ the captured $CO_2$ amount ($CC_{CO2}$ [mol/g (cat.)]) is obtained, and
- the hatched area in Fig. 4C divided by the weight of the sample acetate used in the reactor represents an amount produced ($n_i$) of a chemical component i from $t_2$ to $t_3$ per sample weight and thus in synthesizing methanol in the flow reactor with the flow of a gas containing the hydrogen.

Same sample amounts are used in each of the measurements illustrated with FIGS. 4A to 4C. With corresponding measurements, monitoring and parameters a percentage of conversion of $CO_2$ ($X_{CO_2}$) is calculated with the following formula:

$$X_{CO_2} = \frac{\sum n_{i, \ i= CH_3OH, \ CO, CH_4 \ and \ CH_3OCH_3)}}{CC_{CO_2}}$$

With corresponding measurements, monitoring and parameters a percentage of selectivity ($S_i$) can be calculated from an amount ($n_i$) of a produced chemical component i, related to amounts of produced chemical components, like in case of the chemical component $CH_3OH$ with the following formula:

$$S_{CH_3OH} = \frac{n_{CH_3OH}}{\sum n_{i, \ i= CH_3OH, \ CO, CH_4 \ and \ CH_3OCH_3)}}$$

With the above-indicated measurements and performance evaluation and per gram of a respective sample used in the flow reactor amounts of captured $CO_2$ (converted into g/g (cat.)), amounts of produced $CH_3OH$ (converted into mmol/g (cat.) and mg/g (cat.)) as well as amounts of produced $CH_3OH$ and hourly amounts of produced $CH_3OH$ (in mg/g (cat.) h) were obtained with values as follows:

| Sample | $CO_2$ [g/g (cat.)] | $CH_3OH$ [mmol/g (cat.)] | $CH_3OH$ [mg/g (cat.)] | $CH_3OH$ [mg/g (cat.) h] |
|---|---|---|---|---|
| acetate | 0.0085 | 3.29E-02 | 1.05 | 0.528 |
| nitrate | 0.0047 | 2.61E-02 | 0.83 | 0.418 |

(continued)

| Sample | CO$_2$ [g/g (cat.)] | CH$_3$OH [mmol/g (cat.)] | CH$_3$OH [mg/g (cat.)] | CH$_3$OH [mg/g (cat.) h] |
|---|---|---|---|---|
| chloride | 0.0026 | 1.65E-06 | 0.05 | 0.026 |

With the above-indicated measurements and performance evaluation and for respective samples used in the flow reactor percentages of conversion of CO2 and percentages of selectivities for CH3OH, CO, and CH4 were obtained with values as follows:

| Sample | X$_{CO_2}$ [%] | S$_{CH3OH}$ [%] | S$_{CO}$ [%] | S$_{CH_4}$ [%] |
|---|---|---|---|---|
| acetate | 19.6 | 86.95 | 13.0 | 0 |
| nitrate | 25.2 | 96.23 | 3.76 | 0 |
| chloride | 1.9 | 100 | 0 | 0 |

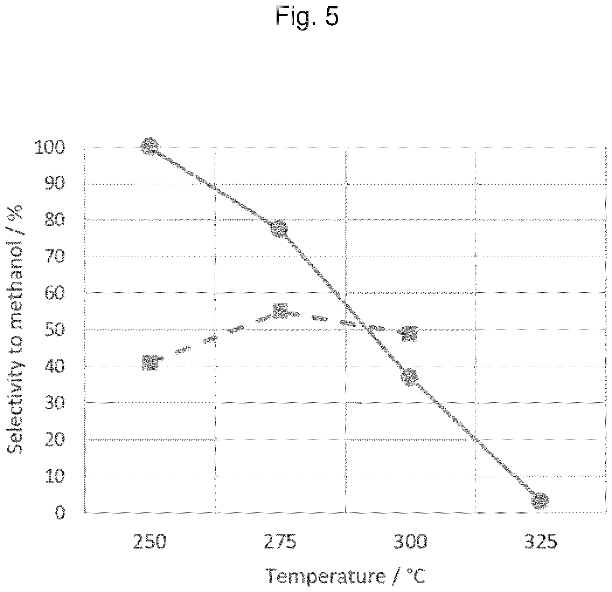

With the sample acetate placed in the tubular fixed-bed reactor the above sequence of gas flows at the pressure of 1 MPa was again established at different temperatures from $t_1$ to $t_3$ as indicated in the graph in FIG. 5 and S$_{CH3OH}$ was determined as indicated above to obtain the tuples indicated with the continuous line in FIG. 5. For the temperatures of 250 °C, 275 °C and 300 °C, these tests were repeated but only until $t_3$ and with replacement of the sequence of gas flows from $t_1$ to $t_3$ by a gas flow composed of 3 vol% CO$_2$ in H$_2$ so as to obtain the tuples indicated with a broken line in FIG. 5. A gas swing operation and method in accordance to the invention (to which the continuous line is FIG. 5 relates) shows higher methanol selectivity at lower temperatures, in particular with a catalyst of the invention, compared to a method for synthesizing methanol from carbon dioxide in a continuous gas flow (without gas flow replacement and to which the broken line in FIG. 5 relates).

Although the present disclosure refers to specific exemplary embodiments, modifications may be provided to these examples without departing from the general scope of the invention as defined by the claims. In particular, individual characteristics of the different illustrated/mentioned sample and related process sequences may be combined in additional aspects. Therefore, the description and the drawings should be considered in an illustrative rather than in a restrictive sense.

**Claims**

1. A catalyst for synthesizing methanol from carbon dioxide and hydrogen via a catalysis, comprising a zirconium oxide support and catalytic sites provided on the support for the catalysis and from an impregnation with a precursor solution for forming zinc oxide crystals, wherein
amounts of zinc atoms and zirconium atoms determined on the catalyst by XPS show a ratio of an amount of zinc to an amount of zirconium of 0.4 or more.

2. The catalyst according to claim 1, wherein a material of the support has a monoclinic crystal structure.

3. A method of producing the catalyst according to claim 1 or 2, comprising steps of

providing a dry material of the zirconium oxide support,
impregnating the dry material with a precursor for providing catalytic sies of zinc via impregnation in which zinc is associated and/or interacts with an organic component, and
removing solvent from the aqueous solution.

4. The method according to claim 3, wherein the precursor is an aqueous solution of a carboxylic acid salt of zinc.

5. The method according to claim 3 or 4, wherein the dry material is a powder.

6. The method according to claim 5, wherein the step of impregnating includes an operation of dropping the aqueous solution at 0.5 to 1 ml/second onto the support heated to a temperature between 80 °C and 100 °C.

7. The method according to claim 6, wherein the step of impregnating includes the operation of dropping alternately with an operation of grinding for obtaining a powder.

8. The catalyst according to claim 1 or 2 and obtainable by the method according to any one of claims 3 to 7.

9. The catalyst according to claims 2 and 8, wherein
in an X-ray diffraction measurement, a ratio of an intensity of a peak attributable to zinc oxide crystals having a hexagonal crystal structure and in a range of Bragg angles between $2\theta = 35°$ and $2\theta = 38°$ to an intensity of a peak attributable to monoclinic zirconium oxide crystals and in a range of Bragg angles between $2\theta = 40°$ and $2\theta = 43°$ is 1.7 or more.

10. A use of an aqueous solution of dissolved zinc(II)carboxylate as the carboxylic acid salt of zinc in the method according to any of claims 3 to 7.

11. A flow reactor for synthesizing methanol from carbon dioxide and hydrogen comprising the catalyst according to any one of claims 1, 2, 8, and 9.

12. A method for synthesizing methanol from carbon dioxide and hydrogen via a catalysis with the catalyst according to any one of claims 1, 2, 8, and 9 in the flow reactor according to claim 11, comprising steps of

establishing in the flow reactor a flow of a gas containing the carbon dioxide for an interaction between the carbon dioxide and the catalyst, and
replacing in the flow reactor the flow of the gas containing the carbon dioxide by a flow of a gas containing the hydrogen and synthesizing methanol in the flow reactor with the flow of a gas containing the hydrogen.

13. The method according to claim 12, wherein the catalysis proceeds at a temperature between 180 °C and 400 °C and a pressure of less than 1.5 MPa.

14. The method according to claim 12 or 13, wherein the gas flow including carbon dioxide constitutes an inert atmosphere with a concentration of carbon dioxide of 20 vol% or less.

15. A use of the catalyst according to any of claims 1, 2, 8, and 9 as a catalyst for synthesizing methanol from carbon dioxide and hydrogen in the method according to any of claims 12 to 14.

**Amended claims in accordance with Rule 137(2) EPC.**

1. A method of producing a catalyst for synthesizing methanol from carbon dioxide and hydrogen via a catalysis, comprising a zirconium oxide support and catalytic sites provided on the support for the catalysis and from an impregnation with a precursor solution for forming zinc oxide crystals, wherein

amounts of zinc atoms and zirconium atoms determined on the catalyst by XPS show a ratio of an amount of zinc to an amount of zirconium of 0.4 or more,
the method comprising steps of
providing a dry material of the zirconium oxide support,
impregnating the dry material with a precursor for providing catalytic sies of zinc via impregnation, and
removing solvent from the aqueous solution,
**characterized in that**
the precursor is an aqueous solution of a carboxylic acid salt of zinc.

2. The method according to claim 1, wherein the dry material is a powder.

3. The method according to claim 1 or 2, wherein the step of impregnating includes an operation of dropping the aqueous solution at 0.5 to 1 ml/second onto the support heated to a temperature between 80 °C and 100 °C.

4. The method according to claim 3, wherein the step of impregnating includes the operation of dropping alternately with an operation of grinding for obtaining a powder.

5. A catalyst obtainable by the method according to any one of claims 1 to 4.

6. The catalyst according to claim 5, wherein a material of the support has a monoclinic crystal structure.

7. The catalyst according to claim 5 or 6, wherein
in an X-ray diffraction measurement, a ratio of an intensity of a peak attributable to zinc oxide crystals having a hexagonal crystal structure and in a range of Bragg angles between $2\theta = 35°$ and $2\theta = 38°$ to an intensity of a peak attributable to monoclinic zirconium oxide crystals and in a range of Bragg angles between $2\theta = 40°$ and $2\theta = 43°$ is 1.7 or more.

8. A flow reactor for synthesizing methanol from carbon dioxide and hydrogen comprising the catalyst according to any one of claims 5, 6, and 7.

9. A method for synthesizing methanol from carbon dioxide and hydrogen via a catalysis with the catalyst according to any one of claims 5, 6, and 7 in the flow reactor according to claim 8, comprising steps of

establishing in the flow reactor a flow of a gas containing the carbon dioxide for an interaction between the carbon dioxide and the catalyst, and
replacing in the flow reactor the flow of the gas containing the carbon dioxide by a flow of a gas containing the hydrogen and synthesizing methanol in the flow reactor with the flow of a gas containing the hydrogen.

10. The method according to claim 9, wherein the catalysis proceeds at a temperature between 180 °C and 400 °C and a pressure of less than 1.5 MPa.

11. The method according to claim 9 or 10, wherein the gas flow including carbon dioxide constitutes an inert atmosphere with a concentration of carbon dioxide of 20 vol% or less.

## FIG. 1A

## FIG. 1B

Fig. 2

# Fig. 3

FIG. 4A

$t_0$      $t_1$   $t_2$

FIG. 4B

$t_0$      $t_1$   $t_2$

FIG. 4C

$t_2$      $t_3$   $t_4$

Fig. 5

Europäisches
Patentamt

European
Patent Office

Office européen
des brevets

# EUROPEAN SEARCH REPORT

**Application Number**

EP 24 21 5169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TADA SHOHEI ET AL: "Active Sites on Zn x Zr 1- x O 2- x Solid Solution Catalysts for CO 2 -to-Methanol Hydrogenation", ACS CATALYSIS, vol. 12, no. 13, 20 June 2022 (2022-06-20), pages 7748-7759, XP093267000, US ISSN: 2155-5435, DOI: 10.1021/acscatal.2c01996 Retrieved from the Internet: URL:https://pubs.acs.org/doi/pdf/10.1021/a cscatal.2c01996?casa_token=Ngnpnl10JiYAAAA A:sDlDqoIXLNuhwjzahjbrNwAID1Sfug-zqEbwgOXs VcY_aCNgUWiIpbBDTOQqqf3oFsa5X4Ht4wXkaMXU> | 1,3,5,8, 9,11 | INV. B01J21/06 B01J23/06 B01J35/70 B01J37/02 C07C29/153 |
| Y | * 2. Methods; | 2 | |
| A | figure 3; table 1 * | 4,6,7, 10,12-15 | |

-----

-/--

|  |
|---|
| TECHNICAL FIELDS SEARCHED (IPC) |
| B01J C07C |

The present search report has been drawn up for all claims

| Place of search | Date of completion of the search | Examiner |
|---|---|---|
| Munich | 7 April 2025 | Mauger, Jeremy |

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | NIKOLAJSEN M.T. ET AL: "Surface ZnO on zirconia is highly active for high temperature methanol synthesis", JOURNAL OF CATALYSIS, vol. 431, 1 March 2024 (2024-03-01), page 115389, XP093267035, US ISSN: 0021-9517, DOI: 10.1016/j.jcat.2024.115389 Retrieved from the Internet: URL:https://pdf.sciencedirectassets.com/272561/1-s2.0-S0021951724X00032/1-s2.0-S0021951724001027/main.pdf?hash=edb9a68feb3ebd897edca2f469e878c8c3dc1f3c9a049108e3747fa0ee269a0d&host=68042c943591013ac2b2430a89b270f6af2c76d8dfd086a07176afe7c76c2c61&pii=S0021951724001027&tid=spdf-532b4955-8f07-49fa-8238-dbf> * 2. Materials and Methods, 3.1. Support effects in ZnO catalysed CO2 hydrogenation; figure 1 * ----- | 2 | |
| A | CN 117 427 626 A (UNIV SCIENCE & TECHNOLOGY CHINA) 23 January 2024 (2024-01-23) * claims * ----- | 1-15 | **TECHNICAL FIELDS SEARCHED (IPC)** |

The present search report has been drawn up for all claims

| Place of search | Date of completion of the search | Examiner |
|---|---|---|
| Munich | 7 April 2025 | Mauger, Jeremy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................................

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 21 5169

This annex lists the patent family members relating to the patent documents cited in the above-mentioned European search report.
The members are as contained in the European Patent Office EDP file on
The European Patent Office is in no way liable for these particulars which are merely given for the purpose of information.

07-04-2025

| Patent document cited in search report | Publication date | Patent family member(s) | Publication date |
|---|---|---|---|
| CN 117427626 A | 23-01-2024 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108940254 A
- CN 116328779 A
- CN 116328773 A
- CN 113145113 A

**Non-patent literature cited in the description**

- **XU et al.** Highly dispersed metal doping to ZnZr oxide catalyst for CO2 hydrogenation to methanol: Insight into hydrogen spillover. *J. Catal.*, 2021, vol. 393, https://doi.org/10.1016/j.jcat.2020.11.039
- **LEE et al.** Atomic Pd-promoted ZnZrOx solid solution catalyst for CO2 hydrogenation to methanol. *Appl. Catal. B Environ.*, 2022, vol. 304, https://doi.org/10.1016/j.apcatb.2021.120994
- **FENG et al.** Asymmetric Sites on the ZnZrOx Catalyst for Promoting Formate Formation and Transformation in CO2 Hydrogenation. *J. Am. Chem. Soc.*, 2023, vol. 23 (0), 145, https://doi.org/10.1021/-jacs.3c02248
- **HUSSAIN et al.** First principles investigations of structural and electronic properties of Ga-doped ZnZrOx solid solutions for catalytic reduction of CO. *Mol. Cat.*, 2023, vol. 537, https://doi.org/10.1016/j.mcat.2023.112941
- **TEMVUTTIROJN et al.** Role of Calcination Temperatures of ZrO2 Support on Methanol Synthesis from CO2 Hydrogenation at High Reaction Temperatures over ZnOx/ZrO2 Catalysts. *Ind. Eng. Chem. Res.*, 2020, vol. 13 (0), 59, https://doi.org/10.1021/acs.iecr.9b05691

- **REDEKOP et al.** Zn Redistribution and Volatility in ZnZrOx Catalysts for CO2 Hydrogenation. *Chem. Mater.*, 2023, vol. 24 (0), 35, https://doi.org/10.1021/acs.chemmater.3c01632
- **SHA et al.** The role of surface hydroxyls on ZnZrOx solid solution catalyst in CO2 hydrogenation to methanol. *Chin. J. Catal.*, 2023, 45, https://doi.org/10.1016/S1872-2067(22)64176-7
- **MAO et al.** The lattice oxygen determines the methanol selectivity in CO2 hydrogenation over ZnZrOx catalysts. *Catalysis Science & Technology*, 2023, vol. 2 (0), 14, https://doi.org/10.1039/d3-cy01471a
- **WANG et al.** A highly selective and stable ZnO-ZrO2 solid solution catalyst for CO2 hydrogenation to methanol.. *Sci Adv.*, 06 October 2017, vol. 3 (10), e1701290
- **ZOU et al.** Design of technical ZnO/ZrO2 catalysts for CO2 hydrogenation to green methanol. *J. Catal.*, 2024, vol. 430, 115344
- **NIKOLAJSEN et al.** Surface ZnOX on zirconia is highly active for high temperature methanol synthesis. *J. Catal.*, 2024, vol. 431, 115389